(19) Europäisches Patentamt
European Patent Office
Office européen des brevets

(11) Veröffentlichungsnummer: **0 224 772 A1**

(12) # EUROPÄISCHE PATENTANMELDUNG

(21) Anmeldenummer: 86115788.1

(22) Anmeldetag: 13.11.86

(51) Int.Cl.⁴: A 01 N 47/36
C 07 D 333/38

(30) Priorität: 26.11.85 DE 3541631

(43) Veröffentlichungstag der Anmeldung:
10.06.87 Patentblatt 87/24

(84) Benannte Vertragsstaaten:
DE FR IT

(71) Anmelder: BAYER AG
Konzernverwaltung RP Patentabteilung
D-5090 Leverkusen 1 Bayerwerk(DE)

(72) Erfinder: Hallenbach, Werner, Dr.
Kleiststrasse 10
D-4018 Langenfeld(DE)

(72) Erfinder: Lindel, Hans, Dr.
Carl-Duisberg-Strasse 321
D-5090 Leverkusen 1(DE)

(72) Erfinder: Brandes, Wilhelm, Dr.
Eichendorffstrasse 3
D-5653 Leichlingen(DE)

(54) **Selektiv-fungizide Verwendung von Thienylharnstoff-Derivaten.**

(57) Selektiv-fungizide Verwendung von Thienylharnstoff-Derivaten der Formel (I)

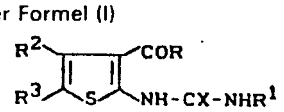

(I)

in welcher

X, R bis $R^3$ die in der Beschreibung angegebenen Bedeutungen haben, gegen Botrytispilze.

EP 0 224 772 A1

0224772

BAYER AKTIENGESELLSCHAFT
Konzernverwaltung RP
Patentabteilung

5090 Leverkusen, Bayerwerk
25. NOV. 1985
Bas/Kü-c
IIb

# Selektiv-fungizide Verwendung von Thienylharnstoff-Derivaten

Die vorliegende Erfindung betrifft die Verwendung von Thienylharnstoff-Derivaten als selektives Fungizid gegen Botrytispilze.

Es ist bereits bekannt geworden, daß bestimmte Thienylharnstoffe, wie z. B. 1-(4,5-Dimethyl-3-ethoxycarbonyl-2-thienyl)-3-methylharnstoff, eine gute fungizide Wirksamkeit besitzen (vergl. z. B. US-PS 3 823 161). Über die vom wirtschaftlichen Standpunkt aus hoch interessante Wirksamkeit gegen Botrytispilze ist jedoch nichts bekannt.

Es wurde gefunden, daß speziell die Verbindungen der Formel (I)

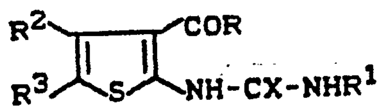 (I)

in welcher

X für Sauerstoff oder Schwefel steht,

R für $C_1$-$C_4$-Alkoxy steht,

$R^1$ für $C_3$-$C_4$-Alkyl steht,

$R^2$ für Wasserstoff oder $C_1$-$C_4$-Alkyl steht und

$R^3$ für Wasserstoff oder $C_1$-$C_4$-Alkyl steht, mit der Maßgabe, daß $R^2$ und $R^3$ nicht gleichzeitig für Wasserstoff stehen,

wegen ihrer besonders guten selektiven fungiziden Wirksamkeit gegen Botrytispilze zur Bekämpfung derselben verwendet werden können.

Überraschenderweise zeigen die Thienylharnstoff-Derivate der Formel (I) eine erheblich höhere selektive fungizide Wirkung gegen Botrytispilze als die aus dem Stand der Technik bekannte Verbindung 1-(4,5-Dimethyl-3-ethoxycarbonyl-2-thienyl)-3-methylharnstoff (vergl. z. B. US-PS 3 823 161).

Der $C_3$-$C_4$-Alkylrest $R^1$ bedeutet geradkettiges oder verzweigtes Alkyl mit 3 oder 4 Kohlenstoffatomen. Beispielhaft seien n-Propyl, i-Propyl, n-Butyl, i-Butyl, sec.-Butyl und tert.-Butyl genannt.

Der $C_1$-$C_4$-Alkoxyrest R bedeutet geradkettiges oder verzweigtes Alkoxy mit 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Die $C_1$-$C_4$-Alkylreste $R^2$ und $R^3$ bedeuten geradkettiges oder verzweigtes Alkyl mit 1 bis 4 Kohlenstoffatomen. Beispielhaft seien Methyl, Ethyl, n-Propyl, i-Propyl, n-Butyl, i-Butyl, sec.-Butyl oder tert.-Butyl genannt.

X steht vorzugsweise für Sauerstoff.

Die erfindungsgemäß zu verwendenden Thienylharnstoff-Derivate sind durch die Formel (I) allgemein definiert. In dieser Formel stehen vorzugsweise

X für Sauerstoff,

R für Methoxy, Ethoxy, n-Propoxy, i-Propoxy, n-Butoxy, i-Butoxy, sec.-Butoxy und tert.-Butoxy,

$R^1$ für n-Propyl, i-Propyl, n-Butyl, i-Butyl, sec.-Butyl und tert.-Butyl,

$R^2$ für Wasserstoff, Methyl, Ethyl, n-Propyl, i-Propyl, n-Butyl, i-Butyl, sec.-Butyl und tert.-Butyl und

$R^3$ für Methyl, Ethyl, n-Propyl, i-Propyl, n-Butyl, i-Butyl, sec.-Butyl und tert.-Butyl, oder

X, R und $R^1$ haben die oben angegebenen Bedeutungen und

$R^2$ steht für Methyl, Ethyl, n-Propyl, i-Propyl, n-Butyl, i-Butyl, sec.-Butyl und tert.-Butyl und

$R^3$ steht für Wasserstoff, Methyl, Ethyl, n-Propyl, i-Propyl, n-Butyl, i-Butyl, sec.-Butyl und tert.-Butyl.

Besonders bevorzugt werden diejenigen Verbindungen der Formel (I) verwendet, in denen

X für Sauerstoff steht,

R für Methoxy, Ethoxy oder n-Propoxy steht,

$R^1$ für i-Propyl, i-Butyl, sec.-Butyl, tert.-Butyl oder n-Butyl steht,

$R^2$ für Wasserstoff, Methyl, Ethyl, n-Propyl oder i-Propyl steht und

$R^3$ für Methyl, Ethyl, n-Propyl oder i-Propyl steht, oder

X, R und $R^1$ die oben angegebenen Bedeutungen haben und

$R^2$ für Methyl, Ethyl, n-Propyl, oder i-Propyl steht und

$R^3$ für Wasserstoff, Methyl, Ethyl, n-Propyl, oder i-Propyl steht.

Ganz besonders bevorzugt werden diejenigen Verbindungen der Formel (I) verwendet, in denen

X für Sauerstoff steht,

R für Ethoxy steht,

$R^1$ für i-Propyl, sec.-Butyl, tert.-Butyl, i- oder n-Butyl steht,

$R^2$ für Wasserstoff, Methyl oder Ethyl steht und

$R^3$ für Methyl, Ethyl oder i-Propyl steht oder

X, R und $R^1$ die oben angegebenen Bedeutungen haben und

$R^2$ für Methyl oder Ethyl steht und

$R^3$ für Wasserstoff, Methyl, Ethyl oder i-Propyl steht.

Die erfindungsgemäß zu verwendenden Wirkstoffe der Formel (I) sind bekannt und/oder lassen sich in einfacher Weise nach bekannten Methoden herstellen (vergl. z. B. EP-OS 4 931, DE-AS 20 40 579, DE-AS 21 22 636 (= US-PS 3 823 161), DE-AS 26 27 935 und eine eigene nicht vorveröffentlichte deutsche Anmeldung P 35 29 24 7.4 vom 16.08.1985). So lassen sich z. B. 2-Amino-thiophen-Derivate mit Isocyanaten oder Isothiocyanaten gemäß dem folgenden Formelschema umsetzen:

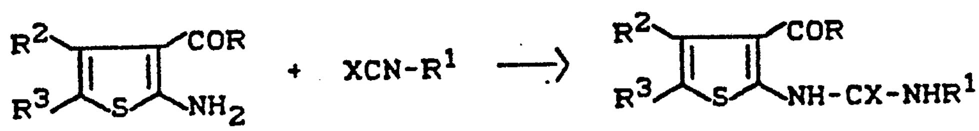

Die Reaktion wird üblicherweise bei Normaldruck und einer Temperatur von 20 °C bis 70 °C durchgeführt, gegebenenfalls in Anwesenheit einer Hilfsbase und in Gegenwart inerter Verdünnungsmittel, wie z. B. Toluol, Chloroform und Pyridin.

Die erfindungsgemäß einzusetzenden Wirkstoffe weisen eine starke selektiv-fungizide Wirkung auf und können zur Bekämpfung von unerwünschten Botrytispilzen praktisch eingesetzt werden. Die Wirkstoffe sind für den Gebrauch im Pflanzenschutz als Botrytismittel geeignet.

Eine besonders gute Wirksamkeit entfalten die erfindungsgemäß einzusetzenden Wirkstoffe z.B. gegen Botrytis cinerea, den Erreger des Grauschimmels an Gartenbohnen, Salat, Erdbeeren und Reben, vorzugsweise werden sie an Reben, Erdbeeren und Salat eingesetzt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw..

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Beispiel A

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik (A) zeigen in diesem Test z. B. die Verbindungen gemäß den Herstellungsbeispielen:
(1), (4), (14), (16) und (18).

## Tabelle A

Botrytis-Test (Bohne)/protektiv

| Wirkstoff | Befall in % bei einer Wirkstoffkonzentration von 100 ppm |
|---|---|
| 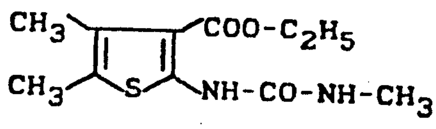 (bekannt) (A) | 29 |
| 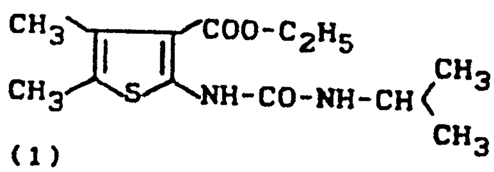 (1) | 2 |
| 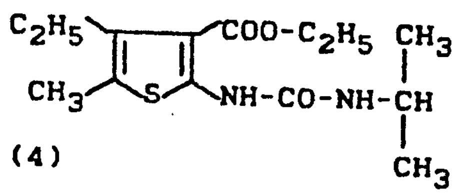 (4) | 2 |
| 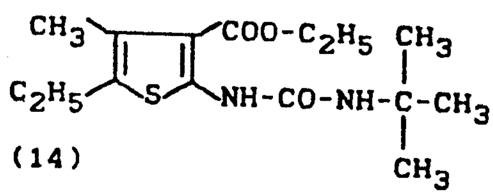 (14) | 6 |

Tabelle A (Fortsetzung)

Botrytis-Test (Bohne)/protektiv

| Wirkstoff | Befall in % bei einer Wirkstoffkonzentration von 100 ppm |
|---|---|
| 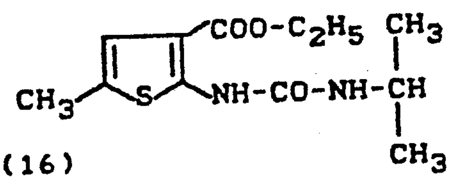 (16) | 8 |
| 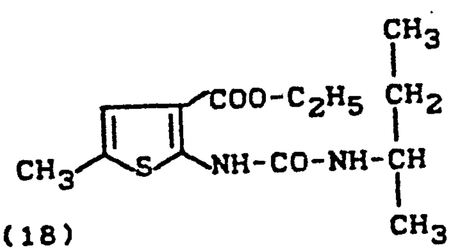 (18) | 7 |

Beispiel B

Botrytis-Test (Rebe)/protektiv

Lösungsmittel: 4,7 Gewichtsteile Aceton
Dispergiermittel: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen:
1, 4, 14, 3, 8 und 9.

## Tabelle B

Botrytis-Test (Rebe)/protektiv

| Wirkstoff | Befall in % bei einer Wirkstoffkonzentration von 25 ppm |
|---|---|
| (bekannt) | |
| 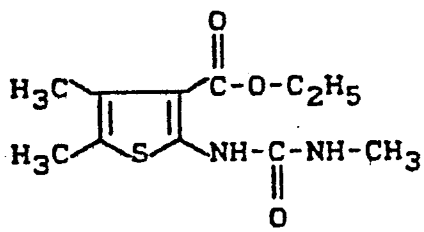 | 32 |
| (erfindungsgemäß) | |
| 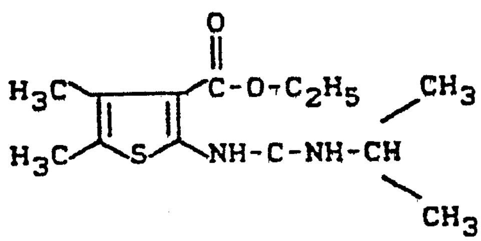 (1) | 14 |
| 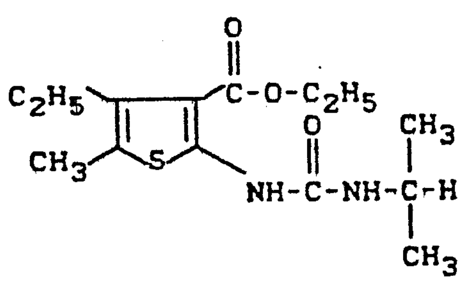 (4) | 5 |
| 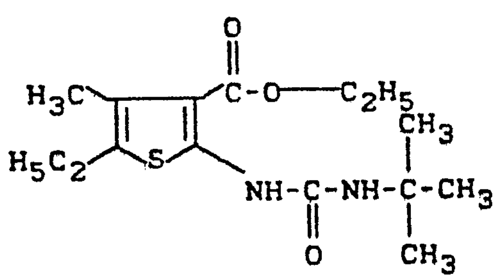 (14) | 24 |

<u>T a b e l l e B</u> (Fortsetzung)

Botrytis-Test (Rebe)/protektiv

| Wirkstoff | Befall in % bei einer Wirkstoffkonzentration von 25 ppm |
| --- | --- |
| 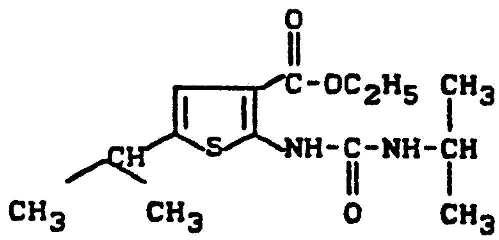 (3) | 10 |
| 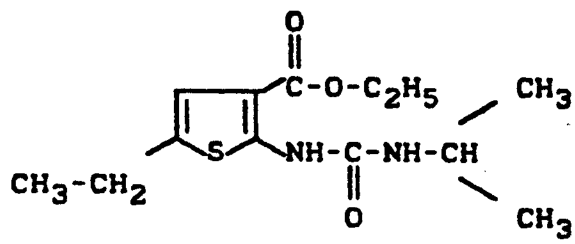 (8) | 10 |
| 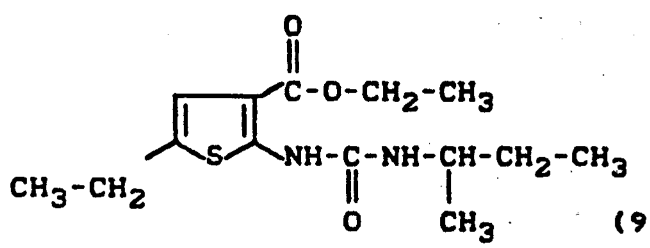 (9) | 7 |

Herstellungsbeispiele

Beispiel 1

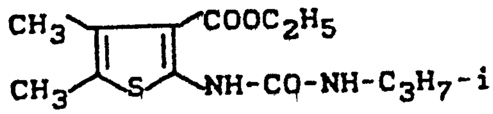

Zu 7 g (0,035 Mol) 2-Amino-3-ethoxycarbonyl-4,5-dimethylthiophen (vergl. Chem. Ber. 99, 94 - 100 (1966)) in 150 ml trockenem Pyridin werden 5 g (0,05 Mol) Isopropylisocyanat zugegeben und 12 Stunden auf 70°C erwärmt. Nach Abkühlung wird in 1 l verdünnte Salzsäure eingerührt, der Niederschlag abgesaugt und aus Ethanol/Wasser umkristallisiert.

Man erhält 5,9 g (59,6 % der Theorie) 1-(4,5-Dimethyl-3-ethoxycarbonyl-2-thienyl)-3-isopropyl-harnstoff vom Schmelzpunkt 135°C.

Analog Beispiel 1 können die folgenden Verbindungen der Formel (I) hergestellt werden:

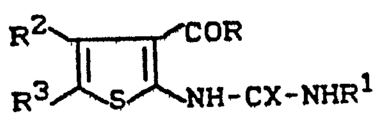

(I)

X = O

Tabelle 1

| Beisp.-Nr. | R | $R^1$ | $R^2$ | $R^3$ | Schmelzpunkt [°C] |
|---|---|---|---|---|---|
| 2 | $-OC_2H_5$ | $-C_4H_9$-n | $-CH_3$ | $-CH_3$ | 78 |
| 3 | $-OC_2H_5$ | $-C_3H_7$-i | H | $-C_3H_7$-i | 91 |
| 4 | $-OC_2H_5$ | $-C_3H_7$-i | $-C_2H_5$ | $-CH_3$ | 139 - 140 |
| 5 | $-OC_2H_5$ | $-C_4H_9$-n | $-C_2H_5$ | $-CH_3$ | 72 |
| 6 | $-OC_2H_5$ | $-C_4H_9$-t | H | $-C_3H_7$-i | 113 - 114 |
| 7 | $-OC_2H_5$ | $-C_4H_9$-s | H | $-C_3H_7$-i | 122 |
| 8 | $-OC_2H_5$ | $-C_3H_7$-i | H | $-C_2H_5$ | 104 |
| 9 | $-OC_2H_5$ | $-C_4H_9$-s | H | $-C_2H_5$ | 109 |
| 10 | $-OC_2H_5$ | $-C_4H_9$-t | H | $-C_2H_5$ | 146 |
| 11 | $-OC_2H_5$ | $-C_4H_9$-t | $-C_2H_5$ | $-CH_3$ | 169 |
| 12 | $-OC_2H_5$ | $-C_4H_9$-s | $-C_2H_5$ | $-CH_3$ | 139 |
| 13 | $-OC_2H_5$ | $-C_3H_7$-i | $-CH_3$ | $-C_2H_5$ | 82 |

0224772

Tabelle 1 - Fortsetzung

| Beisp.-Nr. | R | $R^1$ | $R^2$ | $R^3$ | Schmelzpunkt [°C] |
|---|---|---|---|---|---|
| 14 | $-OC_2H_5$ | $-C_4H_9$-t | $-CH_3$ | $-C_2H_5$ | 152 |
| 15 | $-OC_2H_5$ | $-C_4H_9$-s | $-CH_3$ | $-C_2H_5$ | Öl |
| 16 | $-OC_2H_5$ | $-C_3H_7$-i | H | $-CH_3$ | 112 |
| 17 | $-OC_2H_5$ | $-C_4H_9$-t | H | $-CH_3$ | 140 |
| 18 | $-OC_2H_5$ | $-C_4H_9$-s | H | $-CH_3$ | 118 |
| 19 | $-OC_2H_5$ | $-C_3H_7$-i | $-CH_3$ | H | 121 |
| 20 | $-OC_2H_5$ | $-C_4H_9$-i | $-CH_3$ | H | 92 |
| 21 | $-OC_2H_5$ | $-C_4H_9$-s | $-CH_3$ | H | 87 |
| 22 | $-OC_2H_5$ | $-C_4H_9$-t | $-CH_3$ | H | 137 |

Patentansprüche

1. Selektiv-fungizide Verwendung von Thienylharnstoff-Derivaten der Formel (I)

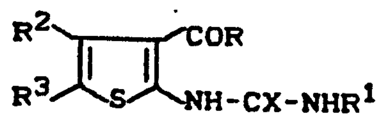

(I)

in welcher

X für Sauerstoff oder Schwefel steht,

R für $C_1$-$C_4$-Alkoxy steht,

$R^1$ für $C_3$-$C_4$-Alkyl steht,

$R^2$ für Wasserstoff oder $C_1$-$C_4$-Alkyl steht und

$R^3$ für Wasserstoff oder $C_1$-$C_4$-Alkyl steht, mit der Maßgabe, daß $R^2$ und $R^3$ nicht gleichzeitig für Wasserstoff stehen,

zur Bekämpfung von Botrytispilzen.

2. Selektiv-fungizide Verwendung von Thienylharnstoff-Derivaten der Formel (I) gemäß Anspruch 1,

in welcher

X für Sauerstoff,

R für Methoxy, Ethoxy, n-Propoxy, i-Propoxy, n-Butoxy, i-Butoxy, s.-Butoxy oder t.-Butoxy,

$R^1$ für n-Propyl, i-Propyl, n-Butyl, i-Butyl, sec.-Butyl oder tert.-Butyl,

$R^2$ für Wasserstoff, Methyl, Ethyl, n-Propyl, i-Propyl, n-Butyl, i-Butyl, s.-Butyl oder t.-Butyl und

$R^3$ für Methyl, Ethyl, n-Propyl, i-Propyl, n-Butyl, i-Butyl, s.-Butyl oder t.-Butyl stehen.

3. Selektiv-fungizide Verwendung von Thienylharnstoff-Derivaten der Formel (I) gemäß Anspruch 1, in welcher

X für Sauerstoff,

R für Methoxy, Ethoxy, n-Propoxy, i-Propoxy, n-Butoxy, i-Butoxy, s.-Butoxy oder t.-Butoxy,

$R^1$ für n-Propyl, i-Propyl, n-Butyl, i-Butyl, s.-Butyl oder t.-Butyl,

$R^2$ für Methyl, Ethyl, n-Propyl, i-Propyl, n-Butyl, i-Butyl, s.-Butyl oder t.-Butyl und

$R^3$ für Wasserstoff, Methyl, Ethyl, n-Propyl, i-Propyl, n-Butyl, i-Butyl, s.-Butyl oder t.-Butyl stehen.

4. Selektiv-fungizide Verwendung von Thienylharnstoff-Derivaten der Formel (I) gemäß Anspruch 1, in welcher

X für Sauerstoff steht,

R für Methoxy, Ethoxy oder n-Propoxy steht,

$R^1$ für i-Propyl, i-Butyl, s.-Butyl, t.-Butyl oder n-Butyl steht,

$R^2$ für Wasserstoff, Methyl, Ethyl, n-Propyl oder i-Propyl steht und

$R^3$ für Methyl, Ethyl, n-Propyl oder i-Propyl steht.

5. Selektiv-fungizide Verwendung von Thienylharnstoff-Derivaten der Formel (I) gemäß Anspruch 1, in welcher

X für Sauerstoff steht,

R für Methoxy, Ethoxy oder n-Propoxy steht,

$R^1$ für i-Propyl, i-Butyl, s.-Butyl, t.-Butyl oder n-Butyl steht,

$R^2$ für Methyl, Ethyl, n-Propyl oder i-Propyl steht und

$R^3$ für Wasserstoff, Methyl, Ethyl, n-Propyl oder i-Propyl steht.

6. Selektiv-fungizide Verwendung von Thienylharnstoff-Derivaten der Formel (I) gemäß Anspruch 1, in welcher

X für Sauerstoff steht,

R für Ethoxy steht,

$R^1$ für i-Propyl, s.-Butyl, t.-Butyl, i- oder n-Butyl steht,

$R^2$ für Wasserstoff, Methyl oder Ethyl steht und

$R^3$ für Methyl, Ethyl oder i-Propyl steht.

7. Selektiv-fungizide Verwendung von Thienylharnstoff-Derivaten der Formel (I) gemäß Anspruch 1, in welcher

X für Sauerstoff steht,

R für Ethoxy steht,

$R^1$ für i-Propyl, s.-Butyl, t.-Butyl, i- oder n-Butyl steht,

$R^2$ für Methyl oder Ethyl steht und

$R^3$ für Wasserstoff, Methyl, Ethyl oder i-Propyl steht.

8. Selektiv-fungizide Verwendung von Thienylharnstoff-Derivaten der Formel (I) gemäß den Ansprüchen 1 bis 7, im Anwendungskonzentrationsbereich zwischen 1 und 0,0001 Gew.-%.

0224772

Europäisches Patentamt

# EUROPÄISCHER RECHERCHENBERICHT

Nummer der Anmeldung

EP 86 11 5788

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| X | JOURNAL OF PHARMACEUTICAL SCIENCES, Band 65, Nr. 5, Mai 1976, Seiten 660-664, Washington, D.C., US; M.B. DEVANI et al.: "Synthesis of 3-substituted thieno[2,3-d]pyrimidin-4(3H)-one-2-mercaptoacetic acids and their ethyl esters for pharmacological screening" * Insgesamt * | 1,8 | A 01 N 47/36<br>C 07 D 333/38 |
| | --- | | |
| A | | 2-7 | |
| | --- | | |
| A | CHEMICAL ABSTRACTS, Band 92, Nr. 9, 3. März 1980, Seite 650, Zusammenfassung Nr. 76205r, Columbus, Ohio, US; V.J. RAM: "Tiophenes, pyrazolothiophenes and pyrimidothiophenes as pesticides", & ARCH. PHARM. (Weinheim, Ger.) 1979, 312(9), 726-33 * Insgesamt * | 1-8 | RECHERCHIERTE SACHGEBIETE (Int. Cl.4)<br>A 01 N |
| | --- | | |
| D,A | DE-A-2 122 636 (ESSO RESEARCH AND ENGINEERING) * Seite 3, Verbindungen 10,22; Seite 10, Zeilen 8-18, Beispiele 27-29; Patentansprüche 1,26-28 * | 1-8 | |
| | --- -/- | | |

Der vorliegende Recherchenbericht wurde für alle Patentansprüche erstellt.

| Recherchenort | Abschlußdatum der Recherche | Prüfer |
| --- | --- | --- |
| DEN HAAG | 11-03-1987 | FLETCHER A.S. |

KATEGORIE DER GENANNTEN DOKUMENTE
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82

0224772

Europäisches Patentamt

# EUROPÄISCHER RECHERCHENBERICHT

Nummer der Anmeldung

EP 86 11 5788

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 004 931 (BAYER) * Seite 24, Zeilen 28-30; Patentansprüche * | 1-8 | |
| D,A | DE-A-2 040 579 (MAY & BAKER) * Seite 16, Zeilen 13-17; Patentansprüche * | 1-8 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 11-03-1987 | FLETCHER A.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument